# EUROPÄISCHE PATENTSCHRIFT

(11) **EP 3 025 742 B1**
(45) Veröffentlichungstag und Bekanntmachung des Hinweises auf die Patenterteilung: **17.01.2018**
(21) Anmeldenummer: 15003334.8
(22) Anmeldetag: 21.11.2015
(51) Int. Cl.: A61M 5/14, A61M 3/02

(54) **TRANSPORTWAGEN FÜR SPÜLLÖSUNGEN**
TRANSPORT TROLLEY FOR FLUSH SOLUTIONS
CHARIOT DE TRANSPORT POUR SOLUTIONS DE RINÇAGE

(30) Priorität: 25.11.2014 DE 102014017397
(43) Veröffentlichungstag der Anmeldung: 01.06.2016
(73) Patentinhaber: Fidica GmbH & Co. KG, 63877 Sailauf (DE)
(72) Erfinder: Völker, Manfred, 63825 Blankenbach (DE)
(74) Vertreter: Flosdorff, Jürgen

(56) Entgegenhaltungen:
- WO-A1-2013/006716
- DE-A1- 4 137 748
- DE-A1- 19 733 278

## Beschreibung

Die Erfindung betrifft einen fahrbaren Spüllösungsbehälter mit einem Transportwagen und einem Druckbehälter, in den ein unter Druck stehendes Gas einbringbar ist, um die Spüllösung aus dem Druckbehälter abzugeben.

Ziel dieser Entwicklung ist es aus Leitungswasser durch den Einsatz von Filtertechnik eine kostengünstige, chemisch und mikrobiologisch hochreine Flüssigkeit als Grundstoff für den Einsatz medizinischer Spüllösungen dezentral vor Ort herzustellen und mit einem Konzentrat so zu mischen, dass größere Volumen gebrauchsfertiger Spüllösungen entstehen, die mobil zum Anwendungsort verbracht und mittels Druckbeaufschlagung verabreicht werden können.

Dabei können sowohl Spüllösungen für endoskopische und allgemein chirurgische Operationen, z.B. Gynäkologie, Urologie, Arthroskopie durch die Verwendung von Purisole-, Ringer-, Kochsalzkonzentraten, als auch Spüllösungen zur Linderung von chronischen Krankheiten bzw. für therapeutische Anwendungen hergestellt werden. Eine Anwendung dieser Entwicklung für andere Bereiche wie z.B. für die Veterinärmedizin, im Labor oder in der Biologie und in der Pharmazie als hochreine Spülflüssigkeit oder auch Ansatzmedium zur Herstellung von Medikamenten, Zellkulturen und dergleichen ist vorstellbar und praktikabel.

Die Erfindung ist dadurch gekennzeichnet, dass die Spüllösung in einen flexiblen Spüllösungsbeutel aufgenommen ist, der in dem Druckbehälter angeordnet ist, dass der Druckbehälter einen Deckel mit einer Konnektoraufnahme hat, durch die ein mit dem Spüllösungsbeutel unlösbar verbundener Beutelkonnektor dicht hindurchführbar ist, der in dem Deckel verriegelbar ist, dass durch den Beutelkonnektor eine Spüllösungsfüllleitung verläuft, dass der Transportwagen eine Behälterwaage aufweist, auf der der Druckbehälter aufliegt, und dass der Transportwagen eine Druckregeleinheit für den Druck in dem Behälter aufweist.

Medizinische Spüllösungen werden in der Regel aus destilliertem Wasser als Grundstoff, der zentral hergestellt wird, in einem zentralen Produktionsprozess zu Spüllösungen weiterverarbeitet und mit erheblichen Logistikkosten zum Anwendungsort gebracht.

Für den medizinischen Einsatz werden beispielsweise industriell hergestellte Spüllösungen mit Volumen 3l, 5l, 10l dem Krankenhaus zur Verfügung gestellt und mit erheblichem innerbetrieblichen, personellen Logistikeinsatz zwischen- u. endgelagert.

Für die Dauer der Operation bzw. Untersuchung reichen diese Beutelvolumina beispielhaft für eine Blasenoperation mit ca. 60 I Spülflüssigkeit nicht aus, so dass ein Springer außerhalb des zentralen OP-Bereiches verfügbar sein muss, um die Beutel bereit zu stellen, aufzuwärmen und zuzureichen.

Die Verabreichung erfolgt teils gravimetrisch oder auch mit Beutel-Druckmanschetten. Zusätzlich sind oft teure Einmalartikel wie beispielsweise Pumpensegmente, oder auch Beutelwärmer erforderlich.
Ein erheblicher Nachteil bei endoskopischen Untersuchungen ist die mangelnde Sicht durch flotierendes Gewebe oder pulsierende Spülflüssigkeit, weil beispielsweise der erforderliche Spülflüssigkeitsdruck zwischen 0,1 bar bis 0,3 bar nicht konstant gehalten wird.

Zur Verbesserung der Wundhygiene ist großzügig zu spülen. Dies verursacht personelle- und auch Materialkosten.

Die regulativen und normativen Anforderungen an die Qualität des Grundstoffes Wassers sind dabei so hoch, dass es bisher nicht möglich ist, vor Ort, z.B. im Krankenhaus, verifizierbare medizinische Spüllösungen auf Bedarf herzustellen. Zum einen sind dies die hohen mikrobiologischen- und zum anderen die erforderlichen chemischen Anforderungen an den Grundstoff Wasser, die einer verifizier- und nachweisbaren normativ geforderten Qualität der vor Ort bedarfsgesteuerten Herstellung entgegenstehen.

Die dezentrale Herstellung von medizinischen Spüllösungen durch Krankenhauspersonal erfordert sichere Abläufe sowohl in der Bedienung, als auch in der Zuverlässigkeit der Technik hinsichtlich der Spüllösungsqualität.

Notwendige Verbesserungen, Zweck und Ziel dieser Erfindung sind deshalb eine kostengünstige, anwenderfreundliche vor-Ort-Herstellung einer Spüllösung mit geringem Personaleinsatz und einem der Untersuchung bzw. auch mehreren Operationen entsprechenden Spülvolumen.

Eine besondere Bedeutung kommt der Rezeptsicherheit, das heißt Einhaltung der vorgeschriebenen Zusammensetzung, - der Homogenität, - Anwendungstemperatur, -Hygiene der Lösung zu.

Dabei sollen eine raumsparende Technik zur Herstellung der Spüllösung - und ein mobiler Spüllösungsbehälter zum Einsatz kommen, der die wesentlichen Komponenten für hohe Hygiene, Sicherheit, einfache Bedienung und einen konstanten Fluss und Druck zur Verabreichung der Spülflüssigkeit enthält.

Eine schwierig zu lösende Aufgabe ist die rasante mikrobiologische Besiedelung innerhalb der Filterstufen bzw. der Wasseraufbereitung dauerhaft zu minimieren bzw. präventiv zu reduzieren. Minimierung und Prävention sind erforderlich, weil die Filterstufen nur eine gewisse Keimrückhaltung von der Primär- zur Sekundärseite aufweisen. Konsequenterweise sind deshalb häufig Wasserproben zu entnehmen und teure mikrobiologische Tests als quasi Revalidierung durchzuführen.

Dabei soll die erforderliche hohe Verfügbarkeit der Geräte bei allen Mess- und Überwachungsaufgaben hinsichtlich ihrer Eigensicherheit nur eine entfernte Ausfallwahrscheinlichkeit aufzeigen, um unter allen Umständen eine katastrophale Auswirkung für den Patienten zu vermeiden, bzw. die Qualität oder auch Toxizität der erzeugten Flüssigkeit einwandfrei in den zugesicherten Akzeptanzkriterien zu überwachen.

Diese Aufgabe wird erfindungsgemäß wirkungsvoll dadurch gelöst, dass zur Herstellung der Spüllösung die Kombination einer Umkehrosmose Membrane und zweier weiterer, Filterstufen beispielsweise Ultra-oder Sterilfilter, bevorzugt als Kapillarmembran, genutzt werden.
Diese Filterkombination und weitere Bestandteile werden nachfolgend als Füllstation bezeichnet.

Zur Herstellung von beispielsweise ca. 60 l gebrauchsfertiger Purisolelösung sind ca. 56l sterilfiltriertes Permeat mit ca. 3,6l hochkonzentrierten Purisolekonzentrat proportional so zu verdünnen bzw. zu mischen, dass die entstandene Spüllösung ohne weitere Prüfungen zur intra- und postoperativen Blasenspülung zur Anwendung kommen kann.

Diese o.a. Spüllösung steht stellvertretend z.B. für Ringer -, und oder andere Natriumchlorid Lösungen, die insbesondere im Bereich der Chirurgie, aber auch in anderen medizinischen bzw. vorgenannten Bereichen eingesetzt werden können. Wobei die Konzentrate und deren Mischungsverhältnisse anzupassen sind.

Um die beschriebenen Nachteile der industriell gefertigten, leicht tragbaren Gebindegrößen zu vermeiden, sind vorrangig Volumen vorgesehen, die dem Anwendungszweck angepasst und damit meist nicht tragbar sind.

Das beschriebene Verfahren und die eingesetzten Komponenten und Volumen sind jedoch nicht darauf reduziert. Bedingt durch die hochreinen Wirkmittel, das exakte Mischen und Verdünnen ist eine große Bandbreite von Spüllösungen herstellbar.

Zur Homogenisierung und Temperierung werden annähernd steriles Permeat erwärmt- und mit zu- dosiertem Konzentrat in einer Mischblock gemischt.
Vor Einleitung in einen sterilen Spüllösungsbehälter-/Beutel erfolgte eine zweite Steril-filtrierung der gemischten Lösung.

Die Proportionierung erfolgt mittels einer Konzentrat- und einer Spüllösungsbehälterwaage,
wobei die Konzentratwaage mit jedem Anhängen des befüllten Konzentrabehälters verfiziert wird.

Gelöst wurde die Aufgabe der sterilen, homogenen und hinsichtlich des Volumens korrekten Befüllung indem mit großem Vorteil ein fahrbarer Spüllösungsbehälter, der vorzugsweise als Druckbehälter ausgebildet ist, mit einem einlegbaren sterilen Spülflüssigkeitsbeutel bestückt wird.
Dazu beinhaltet der fahrbare Spüllösungsbehälter mit Vorteil eine Waage, die den Füllzustand überwacht und die aus Sicherheitsgründen mittels Referenzgewicht automatisch zu testen ist.

Dabei können mittels Elektronik, die über einen aufladbaren Akku betrieben wird, am fahrbaren Spüllösungsbehälter die erforderlichen Parameter als auch deren Abweichungen wie beispielsweise Gewicht, Temperatur und Behälterdruck angezeigt werden.

Indem beispielsweise ein drahtloser Datenaustausch u.a. zwischen der Konzentratwaage in der Füllstation und der Behälterwaage des fahrbaren Spüllösungsbehälters hergestellt wird, erfolgt beispielsweise die Überwachung der Proportionalität und der Temperatur.

Mit großem Vorteil beinhaltet der Spüllösungsbeutel einen unlösbaren Anschlusskonnektor, der durch den verriegelbaren Deckel des Druckbehälters durchgesteckt und fixiert werden kann. Der Anschlusskonnektor ist mit weiterführenden flexiblen Schlauchleitungen versehen, die als Füll- bzw. Transferleitungen ausgebildet sind.

Die geräteseitigen Anschlüsse des Konzentrats- als auch des Spüllösungsbeutels werden an selbstreinigende, verwechslungssichere Anschluss-Konnektoren der Füllstation, die in dieser Anmeldung beispielsweise als Klappenlösungen ausgeführt sind, aber auch geräteseitig als flexible Schlauchleitung ausgeführt sein können, durch den Anwender herbeigeführt.

Zur Verabreichung der Spülflüssigkeit am Anwendungsort wird an den Transferanschluss des Spüllösungskonnektors ein Überleitsystem mit dem Endoskopiesystem verbunden.

Gelöst wurde die Aufgabe der einfachen Bedienung und der Verabreichung mit konstanten Spülfluss und -Drucks indem Druckgas (Luft) entweder vorzugsweise in den Druckbehälter oder auch wahlweise direkt in den Spülflüssigkeitsbeutel eingeleitet wird.

Dabei wird mit Vorteil die Druckgasregelung und Überwachung innerhalb des fahrbaren Spüllösungsbehälters erzeugt. Die Einspeisung kann beispielsweise durch eine hauseigene Quelle, aber auch geräteseitig erzeugt werden.

Die Reinigung des Systems bzw. Keimprävention und Reduktion werden durch die Kombination aus einem gering toxischen, auf Zitrat basierenden Desinfektions- u. Reinigungsmittel mit einer Wassererwärmung ausgeführt. Wobei sowohl die Primär-, als auch die Sekundärseite der Umkehrosmose getrennt voneinander mittels einer zusätzlichen Pumpe auch ohne transmembranen Fluss zu desinfizieren bzw. reinigen sind.

Prinzipiell werden dabei alle prozessrelevanten Daten sowohl vom Betriebs- als auch vom Schutzrechner erfasst und ggf. berechnet. Die Messergebnisse werden vom Betriebs- zum Schutzrechner und umgekehrt gesendet. Jeder Rechner vergleicht dabei die Messergebnisse mit den eigenen und gibt eine Bestätigung zurück.

Die Daten werden nach der Bestätigung von Betriebs- und Schutzrechner zusammen mit einer Prüfsumme in den Trenddatenspeicher geschrieben, der vorzugsweise als Eprom, aber auch als anderes Speichermedium ausgebildet sein kann.

Weitere Einzelheiten und Vorteile sind in den nachfolgend dargestellten Figuren beschrieben.

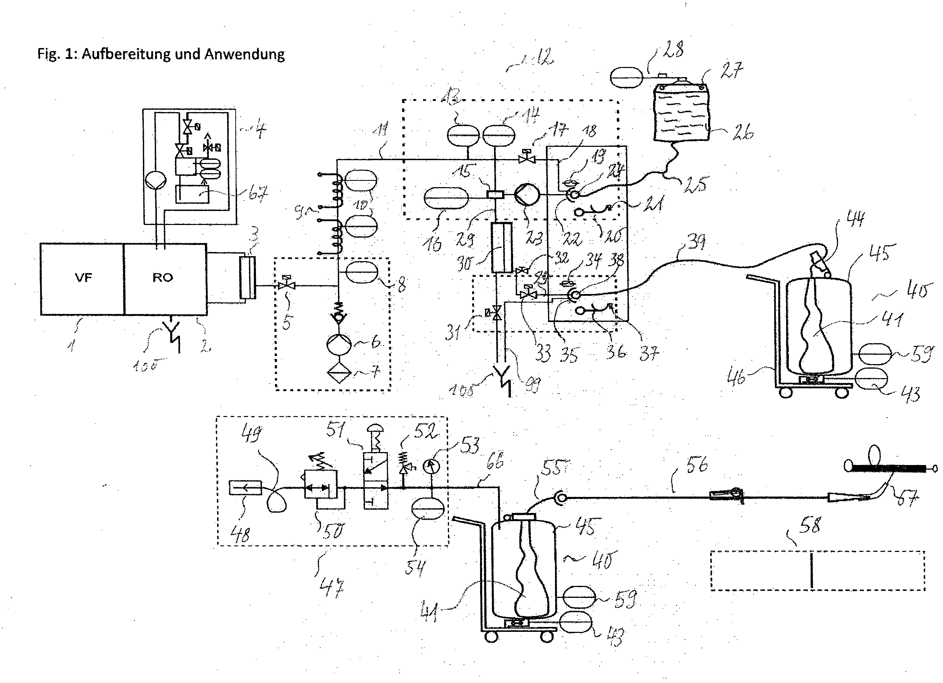

Figur 1 zeigt die gesamte Aufbereitung bis hin zur Anwendung. Die aufzubereitende Flüssigkeit kann beispielsweise über eine optionale Vorfiltration (1), die als Partikel- und oder weiterer Filterstufen zur Eliminierung von Härtebildnern und Chlor ausgebildet sein kann, zur RO-Anlage (2) weitergeleitet werden.
Zur Eliminierung mikrobiologischer Kontamination beinhaltet die RO (2) beispielsweise eine Desinfektionseinheit (4) mit der, ohne Zutun des Anwenders, eine chemothermische Desinfektion durchführbar ist. Kanister (67) beinhaltet das Desinfektions-/Reinigungsmittel das mit Vorteil als citrathaltige Lösung verwendet wird. Die weitere Funktion von Einrichtung (4) leitet sich aus der Darstellung ab und wird hier nicht weiter beschrieben. Selbstverständlich besteht die Möglichkeit einer Heißreinigung der RO-Anlage ohne Verwendung weiterer Desinfektionsmittel.
Das von der RO-Anlage (2) erzeugte Permeat wird über der Primärseite des Filters (3) zirkuliert. Das von der RO-Steuerung (58) mittels nicht dargestellter Leitfähigkeitsmessung freigegebene Permeat gelangt zur Sekundärseite des Filters (3) und über Permeatfreigabeventil (5) zur Mischeinheit (12).
Eventuell bereits von der RO-Anlage (2) vorgewärmtes Permeat wird über Heizer (9) und Temperaturregler (8,13) auf die erforderliche Spüllösungstemperatur erwärmt. Über Leitung (11) wird das Permeat einer Mischkammer (15) zugeführt, in die mittels Pumpe (23) Konzentrat aus Beutel (26) und Leitung (25), dem Konnektor (24) und geräteseitigem Anschlusskonnektor (22) zugeführt wird.
Dabei ist die Konzentratklappe (20) geöffnet, Detektor (19) meldet "offen", weil Magnet (21) die erforderliche Distanz überschritten hat. Das Konzentratspülventil (17) wird nur bei geschlossener Klappe (20) und entsprechend ausgewählten bzw. voreingestellten Spülprogrammen geöffnet, um den Anschlusskonnektor (22) zu reinigen.
Konzentratbeutel (26) ist mit seinen Aufhängungen (27) in entsprechende Haken der Konzentratbeutelwaage (28) eingehängt.
Die zweite Leitfähigkeits- und Temperaturmessung (16) detektiert aus Gründen der Redundanz die entsprechenden Werte. Die durch Kammer (15) homogen gemischte und temperierte Spülflüssigkeit gelangt über Leitung (29) zu einem zweiten Sterilfilter (30).

Fehlerhafte Spülflüssigkeit wird über Bypassventil (31) zum Abfluss (100) verworfen. Bei geschlossenem Ventil (31) und geöffnetem Spüllösungsfreigabeventil (33) wird die Spülflüssigkeit über den geräteseitigen Spüllösungskonnektor (35) den daran konnektierten Beutelkonnektor (38), Leitung (39) zum fahrbaren Spüllösungsbehälter (40) geleitet, in dem ein steriler Spüllösungsbeutel (82) eingelegt ist. Die Möglichkeit zur Entnahme einer Spüllösungsprobenmenge besteht an Probeentnahme (32).

Der fahrbare Spüllösungsbehälter beinhaltet eine Waage (43), die den jeweiligen Füllstand bzw. das Gewicht der Spülmenge registriert. Ebenfalls ist ein Temperaturfühler (59) so angebracht, dass die Spülflüssigkeitstemperaturindirekt messbar ist.

Bei geschlossener Spüllösungsklappe (36) und bei Auswahl und Einleitung eines entsprechenden Spülprogrammes wird der geräteseitige Konnektor (35) mit steriler Flüssigkeit bzw. Reinigungslösung über Spülabfluss (99) gespült bzw. desinfiziert. Der Test der Filter (3/30) erfolgt bei geschlossenen Klappen (20/36) durch gefilterte Luftzuführung mittels Luftpumpe (6) und kann wahlweise durch Ventilschaltung die Sekundärseite des Filters (3) oder die Primärseite des Filters (30) mit Luft beaufschlagen. Dabei wird die Flüssigkeit partiell durch die Luft verdrängt. Durch den hydrophilen Charakter der Filtermembran wird bei intakter Filtercharakteristik nur ein sehr geringer Druckabfall erfolgen, der mittels Drucksensor (14) und Elektronik (58) registriert bzw. überwacht werden kann.
Durch diesen Test können sowohl die Filter (3/30) als auch die Dichtheit der Klappen (20, 36) verifiziert bzw. überprüft werden.

Ebenso ist in Figur 1 schematisch ein möglicher Transfer der Spülflüssigkeit zu einem endoskopischen System (57) dargestellt. Druckluftkonnektor (48) kann mittels flexibler Schlauchleitungen (49) an eine hauseigene Druckgasquelle angeschlossen werden.

Zur Gewährleistung eines konstanten Spülflüssigkeitsflusses beinhaltet die Druckregeleinheit (47) einen einstellbaren Druckregler (50), einen Not-Aus mit Pilztaste und Zwangsentlüftung (51), ein manuelles Druckbegrenzungsventil (52), eine Manometeranzeige (53) und einen elektronischen Druckaufnehmer (54), der wie alle Sensoren und Aktoren mittels redundanter Elektronik (58) ausgewertet und dargestellt werden kann.
Das Niederdruck-Regelventil (50) ist verstellbar. Die Druckregeleinheit (47) kann für einen Regelbereich von 0 bis 0,5bar ausgelegt werden und ist für den praktischen Einsatz auf 0,3 bar Förderdruck beispielsweise bei Prostata Operationen eingestellt. Die so geregelte Luft wird über Schlauchverbindung (66) in Druckbehälter (45) eingeleitet.

Die Spülflüssigkeit in Beutel (41/82) wird durch den zugeführten Druck über Transferanschluss (55) und einem geeigneten Überleitsystem (56) zum endoskopischen System (57) gefördert.

Es versteht sich, dass an System (56) auch andere Einheiten als endoskopische Systeme anschließbar sind.
Zur Ergänzung wird festgestellt, dass an Leitung (55) ein weiterer hier nicht dargestellter Sterilfilter anschließbar wäre.
Ebenso bestünde die Möglichkeit, das geregelte Druckgasmedium direkt im Spüllösungsbeutel (41) einzuleiten.

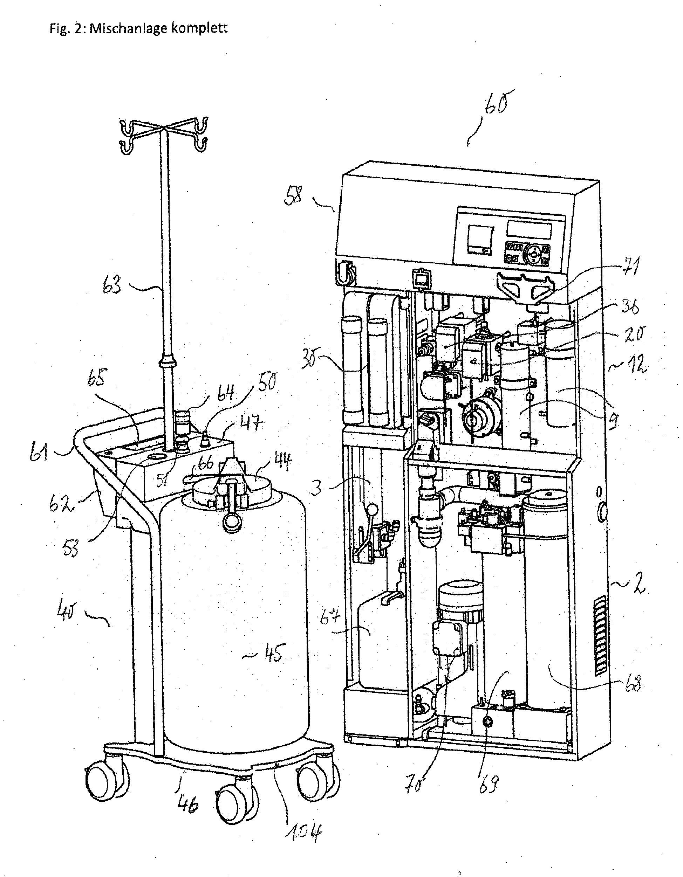

Figur 2 verdeutlicht räumlich die Gesamteinheit einer Mischanlage bzw. Füllstation. Aufgrund der angenommenen räumlich beengten Verhältnisse im Krankenhaus wurde die Füllstation (60) möglichst flach konstruiert um die Durchgänge an Fluren bzw. in Räumlichkeiten nicht zu beeinträchtigen. Dies erfordert eine vertikale Bauweise der RO-Anlage (2) mit Membran (68), Vorlaufbehälter (69) und Pumpe (70). Dargestellt ist auch ein Reinigungskanister (67).
Über der RO-Anlage ist die Mischeinheit (12) angebracht, wobei in dieser Zeichnung lediglich auf die Lage der Konzentratklappe (20), der Spüllösungsklappe (36). dem Heizer (9) und dem Sterilfilter (30) hingewiesen wird, um die Handhabung zu verdeutlichen. Wobei die Klappen hier im geschlossenen Zustand dargestellt sind. Konzentratbeutelwaage (28) ist unterhalb der Elektronik (58) montiert und in Form eines Auslegers (71) mit Aufnahmehaken für den Konzentratbeutel dargestellt.
Die Installation erfolgt wandbündig an geeigneter Stelle mit entsprechendem Höhenabstand zum Fußboden, um Kommunikation - wie später erklärt- und Reinigung zu gewährleisten.

Der fahrbare Spüllösungsbehälter (40) besteht aus einem Transportwagen (46) mit Zug- und Schiebegriff (61), dem Druckbehälter (45), einem Deckel (44), und einer Infusionsstange (63).
Bestandteile des fahrbaren Spüllösungsbehälters (40) sind eine Druckregeleinheit (47), deren Ausgang direkt über eine flexible Schlauchverbindung (66) in den Druckbehälter (45) einmündet und eine Elektronik (62) mit einer Kommunikationsanzeige (65) beispielsweise zur Anzeige des Füllstandes, der Temperatur, Druckluft und anderer relevanter Werte und einer Anzeigeampel (64).

Die Kommunikation zwischen dem Spüllösungsbehälter (40) und der Füllstation (60) erfolgt drahtlos mittels Sensoren im Rollenbereich unterhalb der Bodenplatte (104) des Transportwagens (40).

Die Detektion der Park- bzw. Andockpositionen des Spüllösungsbehälters (40) an die Füllstation (60) ist durch die Lage der vorzugsweisen Infrarotsensoren vorgegeben.

Füllstationsseitig ist auf gleicher Ebene ein korrespondierender Sensor angebracht. Dabei ist durch Auswahl und Lage der Sensoren Andockwinkel und -lage an die Füllstation zu beeinflussen.

Der fahrbare Spüllösungsbehälter (40) kann mit einem Akku und / oder einer Stromversorgung ausgestattet sein; ebenso ist zur Erwärmung bzw. zur verlustfreien Speicherung der erwärmten Spülflüssigkeit eine Isolierung und /oder die Hinzunahme einer Heizung vorzugsweise als Folienheizung möglich. Die Hinzunahme eines internen Kompressors als Druckquelle ist ebenfalls möglich und praktikabel. Ebenso sind auch andere z.B. prismatische Formen einsetzbar.

Die weiteren Komponenten erklären sich teilweise aus der Darstellung oder werden zu einem späteren Zeitpunkt erläutert. Es versteht sich, dass es sich hier um einen raumsparenden Aufbau der Komponenten handelt, deren Anordnung von der dargestellten abweichen kann bzw. auch in anderen Ausführungsformen vorstellbar ist. Ebenso wurde nicht in allen Punkten Bezug auf die Kennzeichnung genommen.

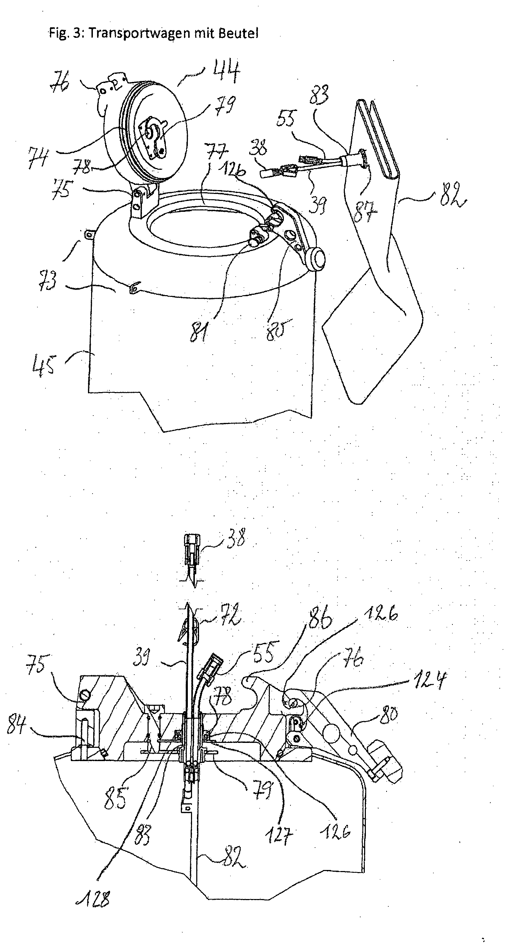

Figur 3 zeigt schematisch den Druckbehälter (45) mit geöffnetem Deckel (44) und einer Konnektoraufnahme (78), durch die der zylindrische Beutelkonnektor (83) eingesteckt wird, und mittels beweglicher Konnektorverriegelung (79) und Haltenut (87) gehalten wird.
Damit es zu einer formschlüssigen Abdichtung mit guten Gleiteigenschaften zwischen Konnektor (83) und Konnektoraufnahmedichtung (78) kommen kann, besteht vorzugsweise Dichtung (78) aus einem Tefloneinsatz (128), der mit einem 0-Ring (126) und einer Druckplatte (127) so gegen Konnektor (83) gepresst wird, dass vorgenannte Ziele erreicht werden.
Eine formschlüssige und dichtende Verbindung des Deckels (44) zum Druckbehälter (45)wird einerseits durch Deckeldichtung (74) und dem konischen Dichtungslager (77) in der Druckbehälteröffnung im geschlossenen Zustand hergestellt.
Zum Verschließen zieht Haken (126) die Deckelverriegelung (76) mittels Verriegelungsgriff (80) in Position. Verriegelungssicherung (81) rastet dabei hinter dem Drehgelenk (124) ein.
Deckelklemmscharnier (75) hält Deckel (44) im geöffneten Zustand in aufrechter Position.

Es versteht sich, dass der Beutel (82) dazu in den Behälter einzubringen ist.
Zur vertikalen Abstützung sind am Druckbehälter (45) zwei seitliche Führungen (73) angebracht.
Die Druckluftzuführung (66) ist beispielsweise im Scharnierbereich (75) mittels Anschluss (84) angebracht.
Konnektorverriegelung (79) ist im Fehlerfalle von außen über Drehwelle (85) mittels Werkzeug zu öffnen.
Ebenfalls ist in dieser Figur die Füllleitung (39) mit Konnektor (38) dargestellt, die im Füllprozess an Anschluss (35) zu konnektieren ist. Nach dem Füllvorgang kann Klemme (72) geschlossen werden. Zur Differenzierung von Füllleitung (39) und Transferleitung (55) sind diese mit unterschiedlichen Konnektoren bestückt und in unterschiedlichen Längen, wie dargestellt, ausgeführt.

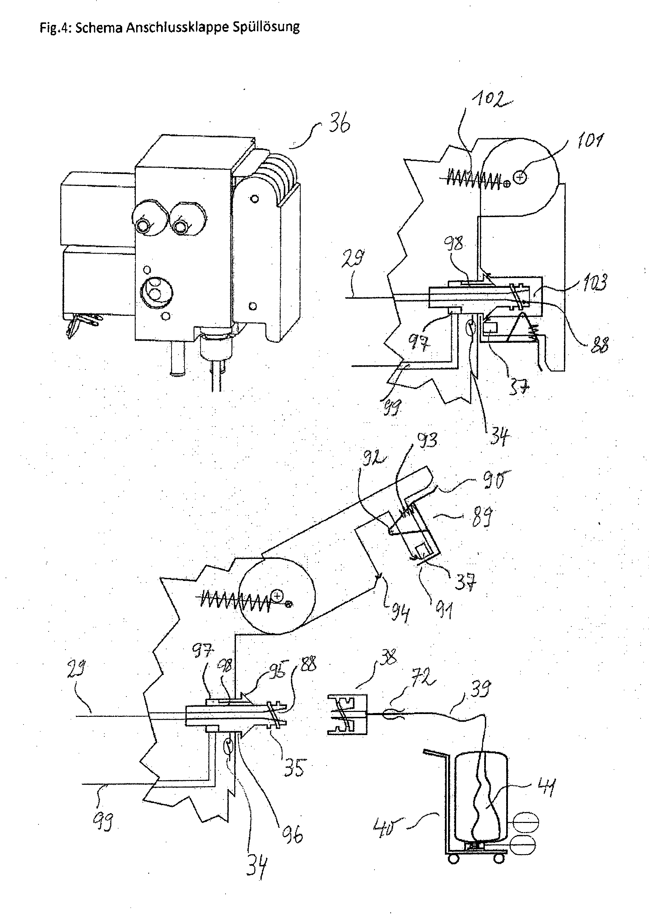

Figur 4 zeigt perspektivisch schematisch die Spüllösungsklappe (36), deren Öffnen, Verschluss, Aushub- und Reinigungsvorgang wie folgt beschrieben wird.

In der Klappe (36) befindet sich ein Magnet (37), der bei geschlossener Klappe einen magnetischen Kontakt (34) aktiviert.
Zum Spülen wird die Klappe (36) geschlossen, sodass der Klappenverriegelungshaken (91) die Klappenverrriegelung (89) in den Verriegelungsbund (96) des Anschlußkonnektors (35) einrastet.

Durch Zurückdrücken der Verriegelung (89) über Drehpunkt (92) mittels Klappenverriegelungsgriff (90) wird die Verriegelungsfeder (93) gestaucht und der Klappenverriegelungshaken (91) gibt dabei den Aushubprozess der Klappe (36) frei. Die Klappe schwenkt nach oben. Unterstützt wird dies durch Aushubfeder (102), die seitlich des Drehpunktes (101) eingreift.
Zur vollständigen Spülung des Konnektors (35) drückt die Dichtung (94) bei geschlossener Klappe formschlüssig auf den Außenkonus (95) des Konnektors (35). Die Spülflüssigkeit gelangt über Anschluss (29) über den Innenkegel (88) zum Spülraum (103) und von dort über die umfangseitig angebrachten Spülbohrungen (98) des Konnektors (35) in den Ringspalt (97), von dem der Spülabfluss (99) erfolgt.

Aus Gründen der Verwechslungssicherheit für die Anwendung wurde die technische Ausführung der Spüllösungsanschlüsse unterschiedlich zu denen der Konzentratanschlüsse konstruiert.

Unter der Spüllösungsklappe (36) befindet sich ein Spüllösungskonnektor (35) ausgeführt beispielsweise mit einem Innenkegel (88) 1 zu 16 und einem zweigängigem Außengewinde 13x8. Am Spüllösungsbeutel (41), der als Einmalartikel ausgeführt ist, befindet sich die Füllleitung (39) mit Einmalkonnektor (38), der als männlicher Konnektor beispielsweise mit frei drehbarer Überwurfmutter mit Innengewinde 13x8 und einem innenliegenden Aussenkegel 1zu16 so ausgestattet ist, dass im gekoppelten Zustand eine formschlüssige, dichtende Verbindung durch die beiden Kegel und Gewinde gewährleistet ist. In die Füllleitung (39) kann eine Schlauchklemme (72) montiert werden.

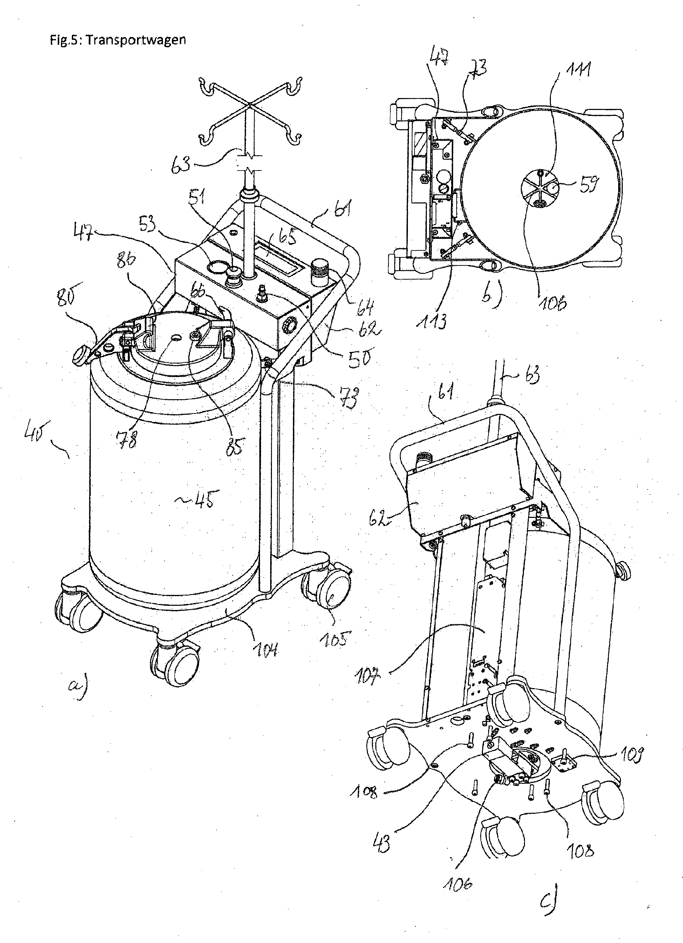

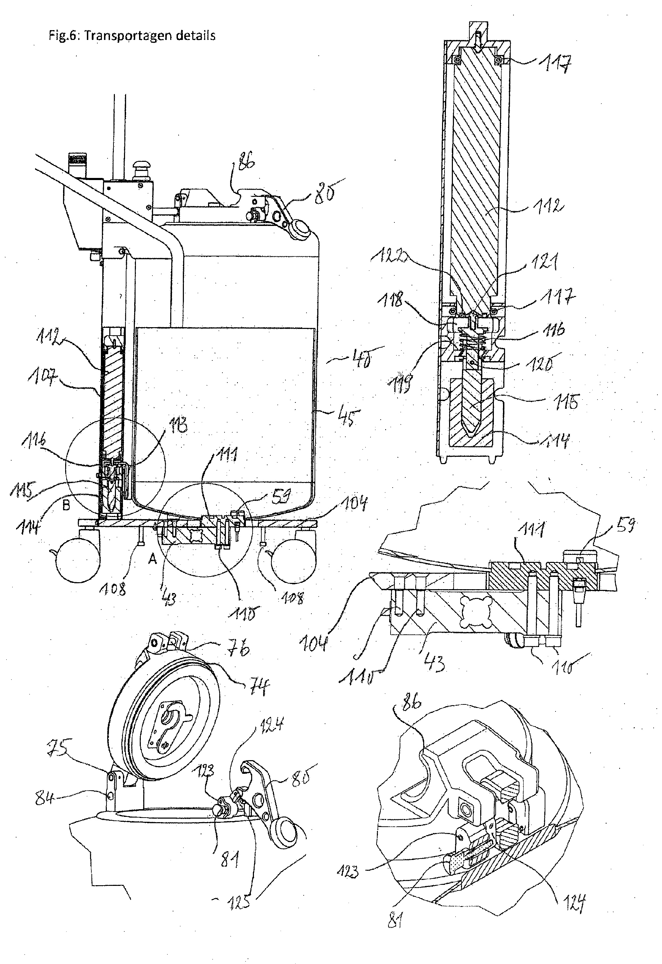

Die Figuren 5 und 6 zeigen weitere Details des Transportwagens (40).
5a stellt perspektivisch den Gesamtzusammenbau vor, dessen Details bereits in vorangegangenen Figuren näher beschrieben wurden.
5b zeigt in einer Schnittdarstellung die obere seitliche Führung (73) des Druckbehälters (45), die als obere Abstützung mit vertikale Spiel dienen. Ebenso sind ein Bodenablauf (106), der in einer Bodenplatte des Druckbehälters (111) eingebracht ist, und die Lage des Temperatursensors (59) dargestellt.

In 5c ist die Befestigung der Spüllösungsbehälterwaage (43) an Bodenplatte (104) und Druckbehälter (111) dargestellt. Zur Absicherung der Waage (43) gegen Transporthindernisse sind die Schrauben/Bolzen (108) vorgesehen.
(109) zeigt die Aufnahme des drahtlosen Kommunikationssensors. (109) kann beispielsweise als Infrarot-Sensor bzw. Sensoren mit entsprechendem transparentem Gehäuse eingesetzt werden.
Füllstationsseitig ist auf gleicher Ebene ein korrespondierender Sensor angebracht. Dabei ist durch Auswahl und Lage der Sensoren Andockwinkel und -lage an die Füllstation zu beeinflussen.

(107) zeigt die Lage einer Waagen-Testeinrichtung, die in Figur 6 näher beschrieben ist.
Die Testeinrichtung (107) besteht aus einem Testgewicht (112), einem Anschlagwinkel (113) und einem Hubmagnet (114). Anschlagwinkel (113) ist an Druckbehälter (45) fest angebracht und greift mit gabelförmigen Ausschnitt in Öffnung (118) ein. Durch Auslösen des Hubmagnets (114) wird Plunger (115) nach unten gezogen, Feder (116) gestaucht, so dass Testgewicht (112) mit Anschlagsdämpfung (122), mit vollem Gewicht auf den Anschlagwinkel (113) drückt, und damit durch Waage (43) registriert werden kann. Nach Beendigung des Testvorganges drückt Hubfeder (116) Testgewicht (122) nach oben und gibt damit den Anschlagwinkel (113) frei.

Die Schrauben (110) zeigen die Befestigung der Wäagezelle (43) an der Bodenplatte (111) des Druckbehälters und der Bodenplatte des fahrbaren Transportwagens (104). Über diese Verbindung ist der Druckbehälter quasi über die Wäagezelle (43) am Fahrgestell bzw. dessen Bodenplatte (104) schwimmend befestigt. Die obere Abstützung mit vertikalem Spiel erfolgt über Halter (73).

Im weiteren Verlauf der Figur wird nochmals die Verriegelung (81) des Verriegelungsgriffes (80) verdeutlicht. Der Verriegelungsgriff (80) ist mittels Drehgelenk (124) an Drehpunkt (123) befestigt. Beim Schließvorgang wird Drehgelenk (124) mit Phase (125) an dem mittels Feder vorgespannten Verriegelungsbolzen (81) vorbei geschwenkt. (81) wird während des Verriegelungsvorganges in eine vorgespannte Stellung gedrückt und schnappt im vollständig verschlossenen Zustand des Deckels (44) hinter dem Drehgelenk (124) ein.

**Legende**

| | |
|---|---|
| 1. | Vorfiltration |
| 2. | RO-Anlage |
| 3. | Permeat Ultra-/Sterilfilter |
| 4. | Desinfektionseinheit |
| 5. | Permeatfreigabeventil |
| 6. | Luftdruckzuführung, Luftpumpe |
| 7. | Luftansaugfilter |
| 8. | Temperaturregler |
| 9. | Heizer |
| 10. | Übertemperaturschutz |
| 11. | Permeatversorgungsleitung |
| 12. | Mischeinheit |
| 13. | Temperaturregler / -anzeige |
| 14. | Drucksensor |
| 15. | Mischkammer |
| 16. | Redundante Leitfähigkeitsmessung / Temperaturanzeige |
| 17. | Konzentratspülventil |
| 18. | Spülleitung |
| 19. | Konzentratklappendetektor |
| 20. | Konzentratklappe |
| 21. | Magnet |
| 22. | Konzentratbeutelanschlusskonnektor mit zweigängigem Innengewinde und innenliegendem Außenkegel |
| 23. | Konzentratpumpe |
| 24. | Konzentratbeutelkonnektor mit Brechkonus mit 2 gängigen Außengewinden und Innenkonus |
| 25. | Konzentratbeutelanschlus |
| 26. | Konzentratbeutel |
| 27. | Aufhängung Konzentratbeutel |
| 28. | Konzentratbeutelwaage |
| 29. | Spüllösungsleitung |
| 30. | Sterilfilter 2 |
| 31. | Spüllösungsbypassventil |
| 32. | Probeentnahme |
| 33. | Spüllösungsfreigabeventil |
| 34. | Spüllösungsklappendetektor |
| 35. | Spüllösungskonnektor mit Innenkegel und zweigängigem Außengewinde |
| 36. | Spüllösungsklappe |
| 37. | Magnet |
| 38. | Spüllösungsbeutelkonnektor mit Außenkonus und Innengewinde |
| 39. | Spüllösungsfüllleitung |
| 40. | Fahrbarer Spüllösungsbehälter |
| 41. | Spüllösungsbeutel |
| 42. | |
| 43. | Spüllösungsbehälter-Waage |
| 44. | Deckel |
| 45. | Druckbehälter |
| 46. | Transportwagen |
| 47. | Druckregeleinheit |
| 48. | Druckluftkonnektor |
| 49. | Schlauchverlängerung |
| 50. | Druckregler |
| 51. | Not-Aus |
| 52. | Druckbegrenzungsventil |
| 53. | Druckmanometer |
| 54. | Drucksensor |
| 55. | Transferanschluss mit zweigängigem Außengewinde, Innenkonus und Verschlussklappe |
| 56. | Überleitsystem |
| 57. | OP-Anwendung |
| 58. | Elektronik |
| 59. | Temperaturfühler |
| 60. | Füllstation |
| 61. | Zug- und Schiebegriff |
| 62. | Elektronik für Transportwagen |
| 63. | Infusionsstange |
| 64. | Anzeige Ampel |
| 65. | Kommunikationsanzeige für Druck, Temperatur, Füllstand |
| 66. | Druckluft-Schlauchverbindung |
| 67. | Desinfektions- / Reinigungsmittelkanister |
| 68. | RO-Membran |
| 69. | Vorlaufbehälter |
| 70. | Pumpe mit Antrieb |
| 71. | Konzentratwaagen-Ausleger mit Beuteleinhänghaken |
| 72. | Schlauchklemme |
| 73. | Seitliche Führung Druckbehälter |
| 74. | Deckeldichtung |
| 75. | Deckelklemmscharnier |
| 76. | Deckelverriegelung |
| 77. | Druckbehälteröffnung mit konischem Dichtungslager |
| 78. | Konnektoraufnahme mit innenliegender vorgespannter Gleitdichtung |
| 79. | Konnektorverriegelung |
| 80. | Verriegelungsgriff mit Haken |
| 81. | Verriegelungssicherung |
| 82. | Spüllösungsbeutel |
| 83. | Beutelkonnektor |
| 84. | Druckluftzuführung |
| 85. | Verriegelungsdrehwelle mit außenliegendem Innensechskant |
| 86. | Aushebegriff |
| 87. | Haltenut |
| 88. | Innenkegel Spüllösungskonnektor |
| 89. | Klappenverriegelung |
| 90. | Klappenverriegelungsgriff |
| 91. | Klappenverriegelungshaken |
| 92. | Klappenverriegelungsdrehpunkt |
| 93. | Klappenverriegelungsfeder |
| 94. | Klappendichtung |
| 95. | Dichtungsgegenlager |
| 96. | Verriegelungsbund |
| 97. | Ringspalt Spülfluss |
| 98. | Spülbohrungen |
| 99. | Spülabfluss |
| 100. | Abfluss |
| 101. | Drehwelle Spüllösungsklappe |
| 102. | Aushubfeder |
| 103. | Spülraum |
| 104. | Bodenplatte fahrbarer Spüllösungsbehälter |
| 105. | Rollen |
| 106. | Abfluss |
| 107. | Waagentesteinrichtung |
| 108. | Anfahrschutz |
| 109. | Drahtlose Kommunikation |
| 110. | Befestigungsschrauben Druckbehälter / Waage |
| 111. | Bodenplatte Druckbehälter |
| 112. | Testgewicht |
| 113. | Anschlagwinkel mit gabelförmiger Aufnahme für Testgewicht |
| 114. | Hubmagnet |
| 115. | Plunger Hubmagnet |
| 116. | Hubfeder für Testgewicht |
| 117. | Führung Testgewicht |
| 118. | Öffnung für Anschlagwinkel |
| 119. | Gegenlager Hubfeder |
| 120. | Tragebolzen Testgewicht |
| 121. | Dämpfungsgummi Tragebolzen |
| 122. | Anschlagsdämpfung |
| 123. | Drehpunkt für Drehgelenk |
| 124. | Drehgelenk |
| 125. | Phase Drehgelenk |
| 126. | O-Ring |
| 127. | Druckplatte |
| 128. | Tefloneinsatz |

## Patentansprüche

1. Fahrbarer Spüllösungsbehälter mit einem Transportwagen und einem Druckbehälter, in den ein unter Druck stehendes Gas einbringbar ist, um die Spüllösung aus dem Druckbehälter abzugeben,
**dadurch gekennzeichnet,**
**dass** die Spüllösung in einen flexiblen Spüllösungsbeutel (82) aufgenommen ist, der in dem Druckbehälter (45) angeordnet ist,
**dass** der Druckbehälter (45) einen Deckel (44) mit einer Konnektoraufnahme (78) hat, durch die ein mit dem Spüllösungsbeutel (82) unlösbar verbundener Beutelkonnektor (83) dicht hindurchführbar ist, der in dem Deckel (44) verriegelbar ist,
**dass** durch den Beutelkonnektor (83) eine Spüllösungsfüllleitung (39) verläuft, dass der Transportwagen (40) eine Behälterwaage (43) aufweist, auf der der Druckbehälter (45) aufliegt, und
**dass** der Transportwagen (40) eine Druckregeleinheit (47) für den Druck in dem Behälter (45) aufweist.

2. Fahrbarer Spüllösungsbehälter nach Anspruch 1,
**dadurch gekennzeichnet,**
**dass** die Behälterwaage (43) an einer Bodenplatte (104) des Transportwagens (40) und einer Bodenplatte (111) des Behälters (45) angebracht ist.

3. Fahrbarer Spüllösungsbehälter nach den Ansprüchen 1 bis 2,
**dadurch gekennzeichnet,**
**dass** an dem Transportwagen (40) ein Testgewicht angeordnet ist, das auf ein Testsignal hin mit dem Behälter verbindbar ist, um dessen Gewicht um das Testgewicht zu erhöhen.

4. Fahrbarer Spüllösungsbehälter nach den Ansprüchen 1 bis 3,
**dadurch gekennzeichnet,**
**dass** an dem Transportwagen (40) eine Infusionsstange (63) befestigt ist.

5. Fahrbarer Spüllösungsbehälter nach den Ansprüchen 1 bis 4,
**dadurch gekennzeichnet,**
**dass** durch den Beutelkonnektor (83) außerdem eine Transferleitung (55) zur Verbindung mit einem Überleitungssystem (56) verläuft.

6. Fahrbarer Spüllösungsbehälter nach den Ansprüchen 1 bis 5,
**dadurch gekennzeichnet,**
**dass** die Spüllösungsfüllleitung (39) und die Transferleitung (55) mit unterschiedlichen Konnektoren bestückt ist.

7. Fahrbarer Spüllösungsbehälter nach den Ansprüchen 1 bis 6,
**dadurch gekennzeichnet,**
**dass** der Transportwagen (40) eine Drucküberwachungseinheit für den Druck in dem Behälter (45) aufweist.

## Claims

1. A mobile flushing solution container with a transport carriage and a pressure container, into which a pressurised gas may be introduced in order to dispense the flushing solution from the pressure container, **characterised in that** the flushing solution is accommodated in a flexible flushing solution bag (82), which is arranged in the pressure container (45), that the pressure container (45) has a cover (44) with a connector receptacle (78), through which a bag connector (83), which is permanently connected to the flushing solution bag (82), and is lockable in the cover (44), that a flushing solution filling conduit (39) extends through the bag connector (83), that the transport carriage (40) includes a container weighing device (43), on which the pressure container (45) rests, and that the transport carriage (40) includes a pressure control unit (47) for the pressure in the container (45).

2. A mobile flushing solution container as claimed in Claim 1, **characterised in that** the container weighing device (43) is mounted on a base plate (104) of the transport carriage (40) and a base plate (111) of the container (45).

3. A mobile flushing solution container as claimed in Claims 1 to 2, **characterised in that** arranged on the transport carriage (40) there is a test weight, which is connectable to the container on a test signal in order to increase its weight by the test weight.

4. A mobile flushing solution container as claimed in Claims 1 to 3, **characterised in that** fastened to the transport carriage (40) there is an infusion rod (63).

5. A mobile flushing solution container as claimed in Claims 1 to 4, **characterised in that** extending through the bag connector (83) there is additionally a transfer conduit (55) for connection with a transfer system (56).

6. A mobile flushing solution container as claimed in Claims 1 to 5, **characterised in that** the flushing solution filling conduit (39) and the transfer conduit (55) are fitted with different connectors.

7. A mobile flushing solution container as claimed in Claims 1 to 6, **characterised in that** the transport carriage (40) includes a pressure monitoring unit for the pressure in the container (45).

## Revendications

1. Récipient de solution de rinçage mobile comportant un chariot de transport et un récipient sous pression, dans lequel un gaz sous pression peut être introduit pour délivrer la solution de rinçage hors du récipient sous pression,
**caractérisé**
**en ce que** la solution de rinçage est reçue dans une poche de solution de rinçage souple (82) qui est disposée dans le récipient sous pression (45),
**en ce que** le récipient sous pression (45) a un couvercle (44) comportant une réception de connecteur (78) au travers de laquelle un connecteur de poche (83) relié de façon non détachable à la poche de solution de rinçage (82) peut être inséré de façon étanche et peut être verrouillé dans le couvercle (44),
**en ce qu'**une conduite de remplissage de solution de rinçage (39) évolue au travers du connecteur de poche (83),
**en ce que** le chariot de transport (40) présente une bascule de récipient (43) sur laquelle repose le récipient sous pression (45), et
**en ce que** le chariot de transport (40) présente une unité de réglage de pression (47) pour la pression dans le récipient (45).

2. Récipient de solution de rinçage mobile selon la revendication 1,
**caractérisé**
**en ce que** la bascule de récipient (43) est aménagée sur une plaque de fond (104) du chariot de transport (40) et une plaque de fond (111) du récipient (45).

3. Récipient de solution de rinçage mobile selon les revendications 1 à 2,
**caractérisé**
**en ce que** sur le chariot de transport (40) est disposée une tare de test qui peut être reliée au récipient sur un signal de test pour augmenter son poids du poids de tare.

4. Récipient de solution de rinçage mobile selon les revendications 1 à 3,
**caractérisé**
**en ce que** sur le chariot de transport (40), une potence à perfusion (63) est fixée.

5. Récipient de solution de rinçage mobile selon les revendications 1 à 4,
**caractérisé**
**en ce que** au travers du connecteur de poche (83), une conduite de transfert (55) évolue en outre pour former une liaison avec un système de transfert (56).

6. Récipient de solution de rinçage mobile selon les revendications 1 à 5,
**caractérisé**
**en ce que** la conduite de remplissage de solution de rinçage (39) et la conduite de transfert (55) sont dotées de différents connecteurs.

7. Récipient de solution de rinçage mobile selon les revendications 1 à 6,
**caractérisé**
**en ce que** le chariot de transport (40) présente une unité de surveillance de pression pour la pression dans le récipient (45).
